# EUROPÄISCHE PATENTSCHRIFT

(11) **EP 2 825 144 B1**
(45) Veröffentlichungstag und Bekanntmachung des Hinweises auf die Patenterteilung: **18.05.2016**
(21) Anmeldenummer: 13708494.3
(22) Anmeldetag: 12.03.2013
(51) Int. Cl.: A61F 13/495, A61F 13/505

(54) **HYGIENEARTIKEL**
SANITARY PRODUCT
ARTICLE D'HYGIÈNE

(30) Priorität: 13.03.2012 DE 102012203860
(43) Veröffentlichungstag der Anmeldung: 21.01.2015
(73) Patentinhaber: Purapur UG (Haftungsbeschränkt), 99425 Weimar (DE)
(72) Erfinder: CLINKSCALES, Angela, 99423 Weimar (DE); DEPTA, Marta, 99423 Weimar (DE)
(74) Vertreter: Kruspig, Volkmar
(86) Internationale Anmeldenummer: PCT/EP2013/055018
(87) Internationale Veröffentlichungsnummer: WO 2013/135711

(56) Entgegenhaltungen:
- EP-A1- 0 911 006
- WO-A1-94/24973
- US-A- 2 690 749
- US-A- 5 795 348
- US-A1- 2006 241 557

## Beschreibung

Die Erfindung betrifft einen Hygieneartikel aus biologisch abbaubaren Materialien, welcher aus einem Innenteil und einem flüssigkeitsabweisenden Außenteil besteht, wobei der Innenteil eine Ausbuchtung zur Aufnahme einer saugfähigen Einlage aufweist, die Einlage lösbar im Innenteil angeordnet ist und das Innenteil integraler Bestandteil des Außenteiles ist, gemäß Patentanspruch 1.

Hygieneartikel zum Aufsaugen und Halten von Urin und anderen Körperausscheidungen sind im Stand der Technik allgemein bekannt. Wegwerfartikel dieser Art werden vorwiegend in den Bereichen Säuglingspflege, Kinderpflege, Damenhygiene und Erwachseneninkontinenz eingesetzt.

Derzeit im Handel erhältliche Wegwerfartikel für derartige Anwendungen sind im Allgemeinen einheitlich, vorgeformt oder vorgefaltet und umfassen eine flüssigkeitsundurchlässige Unterlagsschicht und ein saugfähiges Material, welches zwischen der körperseitigen Einlage und der Unterlagsschicht angeordnet ist.

Auch gibt es sogenannte "Zweistücksysteme", die aus einer bioabbaubaren saugfähigen Einlage und einer wieder verwendbaren, waschbaren äußeren Hülle bestehen.

Aus der Druckschrift US 4,964,857 ist bereits eine Windel bekannt, die über eine herausnehmbare innere Schicht verfügt, welche biologisch abbaubar, sanitär- und umweltfreundlich, ist. Die Windel, entsprechend der Druckschrift US 4,964,857, weist eine "Überhose" auf, in welcher die innere Schicht angeordnet ist, wobei die "Überhose" standardisiert gefertigt ist, so dass sich diese nicht dem Träger anpasst und damit der Tragekomfort stark eingeschränkt ist. Zudem muss die Überhose nach jeder Benutzung entsorgt werden und ist nicht zur Wiederverwendung bestimmt.

Eine wiederverwendbare Windel, die eine Innenschicht aufweist, um Flüssigkeit aufzunehmen und eine Außenschicht, welche flüssigkeitsundurchlässig ist, wobei die Innenschicht von der Außenschicht lösbar und als Einwegmaterial ausgeführt ist, wird in der Druckschrift US 7,629,501 B2 offenbart. Allerdings ist die Innenschicht nach Benutzung nicht ohne Weiteres von der Außenschicht lösbar. Zudem besteht beim Lösen der Innenschicht aus der Außenschicht eine hohe Gefahr, mit den Fäkalien in Kontakt zu kommen.

Auch die Druckschrift US 2011/0137278 A1 offenbart eine Windel, bestehend aus einer äußeren flüssigkeitsabweisenden und einer inneren flüssigkeitsaufnehmenden Schicht, insbesondere Saugkissen, welches aus der äußeren Schicht herausnehmbar ist. Zudem verfügt die innere Schicht über eine zusätzlichen Auslaufschutz in Form eines Winkels. Die Aufnahme von festen Fäkalien ist bei der Windel gemäß der Druckschrift US2011/0137278 nicht vorgesehen.

Die EP 0 911 006 A1 offenbart eine wiederverwendbare Windel mit einer ringförmigen Ausbuchtung mit einer umgebenden Kräuselung in deren Mittelzone, die zu einem sicheren Sammeln von Exkrementen dient.

Die US 2006/0241557 A1 offenbart eine absorbierende Anordnung. Dieser umfasst einen Auslaufschutz mit einem ersten Blatt, einen zweiten Auslaufschutz und einen Rückseitenabschnitt. Die dort genannte Anordnung kann Urin effektiv aufnehmen und ist außerdem zum Trennen von Urin und festen Fäkalien geeignet.

Die US 5,795,348 offenbart eine Windel mit einem sich ausdehnenden Abstandshalter. Dieser sammelt festes Fäkalienmaterial ein. Der Abstandshalter kann von der Windel entnommen werden.

Diese Ausführungsformen einer Windel erlauben zwar eine verbesserte Aufnahme fester Fäkalien. Allerdings zeigt sich bei der praktischen Anwendung sämtlicher Windeln der Nachteil, dass diese Windeln einen nur oftmals ungenügenden Schutz einer Überhose vor grober Verschmutzung gewährleisten, während außerdem die bekannten Anordnungen aus Membranen und Bündchen zu Hautreizungen führen können.

Aus dem Vorgenannten ist es daher Aufgabe der Erfindung einen Hygieneartikel aus biologisch abbaubaren Materialien für Körperflüssigkeiten und festen Fäkalien zu schaffen, der angenehm und komfortabel zu tragen ist und die Nachteile aus dem Stand der Technik beseitigt.

Die Lösung der Aufgabe der Erfindung erfolgt mit der Merkmalskombination gemäß der Lehre nach Patentanspruch 1, wobei die Unteransprüche mindestens zweckmäßige Ausgestaltungen und Weiterbildungen umfassen.

Es wird demgemäß von einem Hygieneartikel aus biologisch abbaubaren Materialien, welcher aus einem Innenteil und einem flüssigkeitsabweisendem Außenteil besteht, wobei der Innenteil eine Ausbuchtung zur Aufnahme einer saugfähigen Einlage, insbesondere eines Saugkissens, aufweist, ausgegangen. Weiterhin ist das Innenteil integraler Bestandteil des Außenteils und die Einlage lösbar im Innenteil angeordnet.

Erfindungsgemäß besitzt die Ausbuchtung eine an die Form der Einlage angepasste Gestaltung und die von der Ausbuchtung aufgenommene Einlage ist dreidimensional-wannenartig ausgeführt, wobei die dreidimensional-wannenartige Form der Einlage, welche auch die Einnahme von festen Fäkalien ermöglicht, durch seitlich angeordnete Saugkissenleisten an der Einlage erzeugt wird. Durch die Flexibilität und Nachgiebigkeit der Saugkissenleisten wird eine dreidimensional-wannenartige Form der Einlage erzielt, die den Träger im Alltag nicht einschränkt.

Im Gegensatz zu den aus dem Stand der Technik bekannten Hygieneartikeln, ist es möglich mit dem erfindungsgemäßen Hygieneartikel neben Körperflüssigkeiten auch feste Fäkalien aufzunehmen. Während die Körperflüssigkeiten vom Saugkissen der Einlage aufgenommen werden, werden die festen Fäkalien in der dreidimensional-wannenartigen Einlage gehalten. Ein Ausfließen der Körperflüssigkeiten und festen Fäkalien in die Außenlage wird verhindert und das Entstehen von Undichtigkeiten des Hygieneartikels abgewendet.

In einer Ausführungsform der Erfindung weist die Ausbuchtung, mindestens im Randbereich, eine Rippung auf. Selbstverständlich ist im Rahmen der Erfindung auch eine vollständige Rippung der Ausbuchtung denkbar. Die erfindungsgemäße Rippung, die bevorzugt elastisch ist, z.B. aus Silikon, kann unterstützend auf die Gestalt der Ausbuchtung und den Halt der Einlage wirken.

In einer weiteren Ausführungsform der Erfindung umschließt die Rippung die Einlage, sobald diese in der Ausbuchtung platziert ist. Dies führt vorteilhafterweise zu einer optimalen Fixierung der Einlage, so dass diese beim Tragen nicht verrutschen kann.

Weiterhin ist die Rippung bevorzugt elastisch z. B. aus Silikon, so dass die Einlage in der Ausbuchtung noch besser fixiert und gehalten werden kann.

Die Ausbuchtung des Außenteils kann sowohl gestrickt sein als auch aus Netzgewebe bestehen. Im Rahmen der Erfindung liegt auch eine Ausbuchtung, die aus verklebten bzw. verschweißten wasserabweisenden Materialien oder Abstandsgewirke besteht.

In einer Ausführungsform der Erfindung weist die Ausbuchtung am Rand eine Einstellvorrichtung auf. Bevorzugt handelt es sich hierbei um einen individuell regulierbaren Gummizug, insbesondere einen Gummi-Kordelzug. Dies ermöglicht eine optimale Anpassung der Ausbuchtung an die saugfähige Einlage und an den jeweiligen Träger. Eine entscheidende Voraussetzung zur Erzielung einer optimalen Funktionalität des Hygieneartikels ist es, die Einlage im Hygieneartikel richtig zu positionieren. Durch den regulierbaren Gummizug ist es möglich, die Einlage in ihrer Position optimal auszurichten. Zudem ist eine der Hauptursachen für das Entstehen von Undichtigkeiten an Hygieneartikeln, das nachträgliche Verschieben der Einlage im Hygieneartikel. Dies wird durch die erfindungsgemäße Einstellvorrichtung am Rand der Ausbuchtung verhindert.

In einer weiteren Ausführungsform handelt es sich bei dem Außenteil des Hygieneartikels um eine Hose, insbesondere um eine Überhose. Dies gewährleistet dem Träger ein einfaches An- und Ausziehen und einen guten Tragekomfort. Durch erfindungsgemäße Verstellmöglichkeiten an der Überhose kann diese an die Größe des jeweiligen Benutzers schnell und einfach angepasst werden.

Weiterhin besteht die Hose bevorzugt aus atmungsaktivem Membranstoffverbund. Hierdurch wird ein angenehmes Tragen erzielt und ein Schwitzen des Trägers minimiert bzw. verhindert.

Entsprechend einer Ausführungsform der Erfindung kann die Hose aus einem oder mehreren Teilen bestehen. Dadurch wird ein flexibles System geschaffen und die Hose ist dem jeweiligen Träger gut anpassbar. Wird die Überhose bzw. das Außenteil des Hygieneartikels aus mehreren Teilen hergestellt, ist es möglich verschiedene Größen der jeweiligen Einzelteile herzustellen und durch individuelle Kombination dieser, eine optimale Anpassung der Überhose an den jeweiligen Träger zu erzielen.

Entsprechend einer Ausführungsform der Erfindung sind die Saugkissenleisten klappbar ausgestaltet. Die dreidimensional-wannenartige Form kann dem Träger individuell angepasst werden und wirkt somit beim Tragen nicht wie ein Fremdkörper. Die erfindungsgemäße Einlage liegt in zweidimensionaler Form vor und wandelt sich erst beim Einbringen der Einlage in die Ausbuchtung des Innenteils des Hygieneartikels in eine dreidimensional-wannenartige Form um. Durch die klappbaren Saugkissenleisten ist die Einlage optimal an die Ausbuchtung des Innenteils anpassbar.

Wie bereits erwähnt, ermöglicht die erfindungsgemäße dreidimensionalwannartige Form der Einlage auch die Aufnahme von festen Fäkalien. Bei den aus dem Stand der Technik bekannten Hygieneartikeln ist die Aufnahme von festen Fäkalien nicht bzw. nur mit großen Unannehmlichkeiten verbunden, möglich. Zudem wird der Auslaufschutz durch die neuartige Form der Einlage wesentlich erhöht. Die Körperflüssigkeiten und festen Fäkalien sammeln sich in der "Wanne" an und können somit einfach entsorgt werden. Ein Übertritt der Körperflüssigkeiten/festen Fäkalien in den Außenteil des Hygieneartikels wird somit verhindert. Eine einfache Entsorgung der Körperflüssigkeiten sowie auch der festen Fäkalien kann gewährleistet werden, so dass auch die Geruchsbelästigung wesentlich eingedämmt wird.

Weiterhin weist die Einlage seitliche Ausformungen auf. Diese sind direkt mit der Einlage verbunden und bestehen in vorteilhafterweise aus dem Grundmaterial der Einlage, wobei dieses in einem Ausführungsbeispiel ein Vliesstoff ist. Durch die seitlichen Ausformungen wird ein sehr dichter Beinabschluss ermöglicht, so dass ein Übertritt der Körperflüssigkeiten nach außen verhindert wird. Die Vliesstoffflügel sind so geschaffen, dass sie sich dem Körper des Trägers anpassen und somit die aus dem Stand der Technik bekannten Durchlässe im Beinbereich minimieren.

In einem weiteren Ausführungsbeispiel sind die seitlichen Ausformungen gerafft. Durch die Raffung der Vliesstoffflügel kann eine zusätzliche Sicherheit beim Träger gewährleistet werden, so dass auch der kritische Beinabschluss, der durch Bewegungen im Alltag besonderen Herausforderungen und Beanspruchungen ausgesetzt ist, einfach abgedichtet wird. Sowohl das Auslaufen von Körperflüssigkeiten in diesem Bereich als auch die Geruchsbelästigung, welche durch Undichtigkeiten entstehen, kann so minimiert werden.

In einer Ausführungsform der Erfindung weist die Einlage einen Saugkern auf, welcher bevorzugt aus mindestens einem Zellstoffkissen besteht.

Weiterhin weisen auch die Saugkissenleisten jeweils einen Saugkern auf, welcher bevorzugt aus mindestens einem Zellstoffkissen besteht.

In einer weiteren Ausführungsform der Erfindung ist in der Einlage, welche einen Saugkern aufweist, eine Prägung eingebracht. Diese Prägung optimiert den Transport der Körperflüssigkeiten in der Einlage. Der Fluss der Körperflüssigkeiten ist somit definiert und ein Austritt der Körperflüssigkeiten in das Außenteil des Hygieneartikels wird verhindert.

Weiterhin weist der Saugkern bevorzugt eine vordefinierte Biegung auf. Diese wird durch die Prägung im Saugkern optimiert. Durch die vordefinierte Biegung des Saugkerns wird verhindert, dass sich die dreidimensional-wannenartige Einlage beim Tragen in die falsche Richtung verbiegt, so dass ein Auslaufen der Körperflüssigkeiten unterbunden wird. Des Weiteren erhöht die vordefinierte Biegung die Flexibilität der Einlage. Die Einlage kann sich somit dem Körper des Trägers anpassen und gewährleistet einen sehr hohen Tragekomfort.

Bei einer zweckmäßigen Ausgestaltung der Erfindung verfügt die Einlage über einem Mechanismus zum Entfernen von festen Fäkalienrückständen. Hierzu verfügt die Einlage über einen mehrschichtigen Aufbau, d. h. eine obere und eine untere Schicht, wobei sich zwischen diesen Schichten der Saugkern befindet.

Die obere Schicht der Einlage verfügt über einen Stoff, insbesondere einen Vliesstoff, der vorteilhafterweise mittels Fäden zusammengezogen werden kann, so dass der Nutzer nicht in Kontakt mit den Körperflüssigkeiten kommt. Das Material der oberen Schicht der Einlage ist dabei so konzipiert, dass dieses nur eine begrenzte Nässefestigkeit für die Dauer der Nutzung aufweist, so dass sich das Material in der Abwasserleitung auflösen kann. Dies ermöglicht eine Minderung der Geruchsbelästigung durch eine Entsorgung der festen Fäkalien in der Toilette, die nicht die Gefahr der Leitungsverstopfung mit sich bringt. Eine benutzerfreundliche Trennung und Entsorgung der Abfälle wird somit gewährleistet.

In einer Ausgestaltung der Erfindung weist das Außenteil ein oder mehrere Verbindungselemente auf. Diese bilden eine lösbare Befestigungsvorrichtung, die die Einlage, insbesondere den Vliesstoff der Einlage, fixiert. Zudem ist die Verbindung gleichzeitig form- und kraftschlüssig. Das Verbindungselement wird aus zwei Kurven mit unterschiedlichem Durchmesser, r1 und r2, gebildet und verfügt zudem über Stopper, die auf dem Verbindungselement aufgebracht sind.

Die Stopper, welche vorzugsweise aus weichem Gummimaterial bestehen, sind in verschiedenen Ausführungen möglich.

Die Erfindung soll nachstehend anhand von Ausführungsbeispielen unter Zuhilfenahmen von Figuren näher erläutert werden.

Hierbei zeigen:
- Fig. 1: Gesamtansicht eines Hygieneartikels mit flüssigkeitsabweisenden Außenteil, integriertem Innenteil sowie mit Einlage
- Fig. 2: Explosionsansicht des Hygieneartikels, Außenteil mit integriertem Innenteil und Einlage
- Fig. 3: Detailansicht der Einlage
- Fig. 4: Schnittansicht A-A der Einlage
- Fig. 5: Detailansicht Einstellvorrichtung der Ausbuchtung
- Fig. 6: Schnittansicht des Hygieneartikels
- Fig. 7: Ansicht der "Überhose"
- Fig. 8: Verbindungselemente in Verbindung mit der Einlage
- Fig. 9: Detailansicht Verbindungselement
- Fig. 10: Stopper in verschiedenen Ausführungsformen

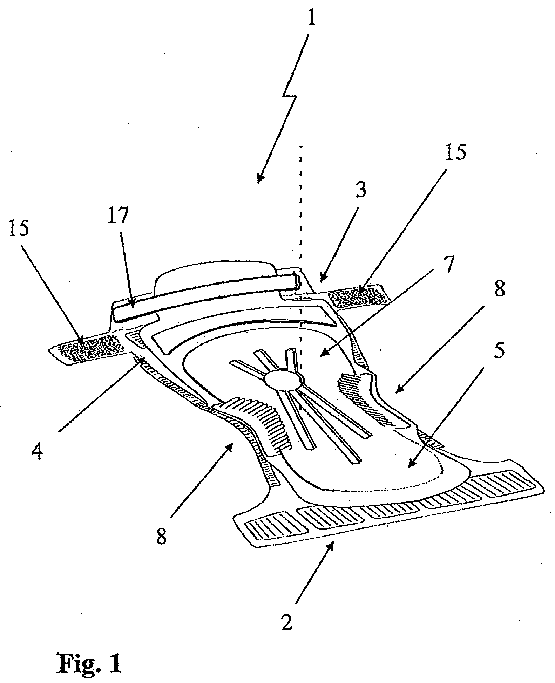

Die Figur 1 zeigt den erfindungsgemäßen Hygieneartikel 1 aus biologisch abbaubaren Materialien, bestehend aus einem Innenteil 5 und einem flüssigkeitsabweisendem Außenteil 4, wobei im Innenteil 5 eine saugfähige Einlage 7 aufgenommen ist. Des Weiteren ist in Figur 1 der Verstellmechanismus 15 dargestellt, der eine individuelle Einstellung der Überhose 4, angepasst an den jeweiligen Träger, ermöglicht. Entsprechend dem Ausführungsbeispiel gemäß Figur 1 handelt es sich bei dem Verstellmechanismus um einen handelsüblichen Klettverschluss 15. Ohne den Rahmen der Erfindung zu verlassen, sind auch andere Verstellmechanismen 15, zur Anpassung der Überhose 4 an den jeweiligen Träger, möglich.

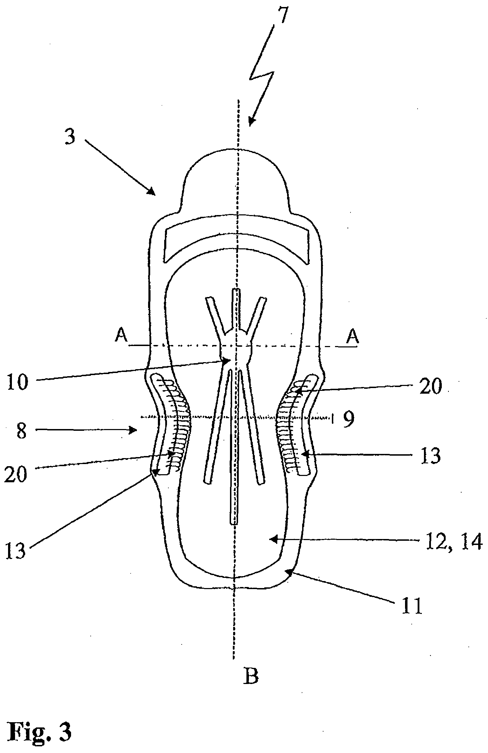

Des Weiteren weist die Überhose 4 einen hinteren Abschnitt 3 und einen vorderen Abschnitt 2 sowie einen dazwischenliegenden Beinabschnitt 8 auf. Die Position des Beinabschnittes 8 des Hygieneartikels 1 befindet sich auf der Höhe der Biegemitte 9 der Einlage 7, siehe Figur 3. Wie in Figur 1 dargestellt, weist der Außenteil 4 der Überhose 4 sowie die Einlage 7 beim Beinabschnitt 8 zudem eine Einkerbung auf, so dass ein guter Tragekomfort erzielt werden kann.

Die Verbindungselemente 17 zur Fixierung der Einlage 7 befinden sich im Ausführungsbeispiel gemäß Figur 1, am hinteren Abschnitt 3, innenseitig, des Außenteiles 4 des Hygieneartikels 1. Allerdings können diese, im Rahmen der Erfindung, auch am vorderen Abschnitt 2 bzw. am hinteren 3 und vorderen Abschnitt 2 angeordnet sein.

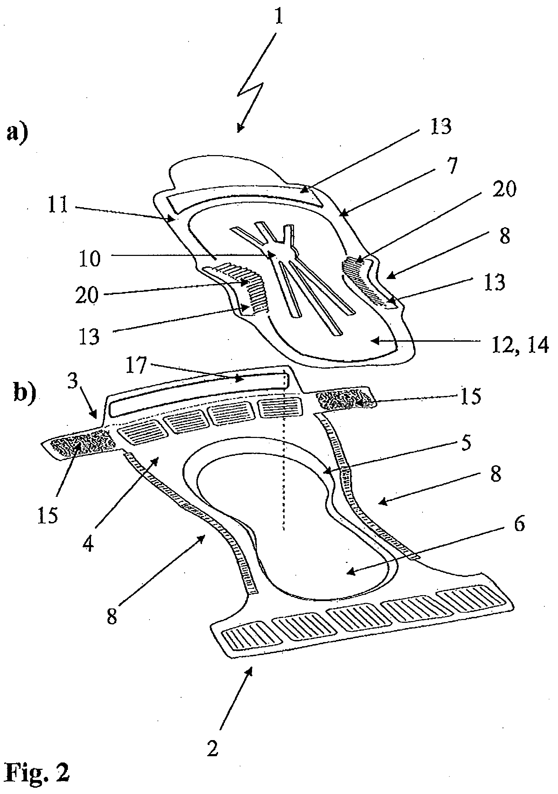

In Figur 2 ist eine Explosionsansicht des erfindungsgemäßen Hygieneartikels 1 dargestellt, wobei in Figur 2a die Einlage 7 und in Figur 2b das Außenteil 4 mit integriertem Innenteil 5, welches eine Ausbuchtung 6 zur Aufnahme der Einlage 7 aufweist, erkennbar ist. Die Ausbuchtung 6 des Innenteils 5 hat eine an die Form der Einlage 7 angepasste Gestaltung.

Die saugfähige Einlage 7 ist in Figur 3 im Detail dargestellt. Die Einlage 7 verfügt über eine Prägung 10, welche eine vordefinierte Biegung der Einlage 7 ermöglicht, so dass sich die erfindungsgemäße dreidimensional-wannenartige Form der Einlage 7, bei Bewegungen des Trägers, nicht in die "falsche" Richtung verbiegt.

Zudem ist in Figur 3 die Biegemitte 9 der Einlage 7 ersichtlich, welche sich auf der Höhe des Beinabschnittes 8 befindet.

Wie bereits erläutert, ist die Einlage 7 dreidimensional-wannenartig ausgeführt, wobei dies durch Saugkissenleisten 13 erzielt wird. Die Saugkissenleisten 13 sind klappbar ausgestaltet, so dass sich die dreidimensional-wannenartige Form der Einlage 7, der Ausbuchtung 6 des Innenteiles 5 und dem Träger anpassen kann.

Entsprechend dem Ausführungsbeispiel gemäß Figur 3 sind drei solcher Saugkissenleisten 13 vorgesehen. Ohne den Rahmen der Erfindung zu verlassen, können auch mehrere oder weniger Saugkissenleisten 13 vorgesehen werden. Die Saugkissenleisten 13 sollten vorteilhafterweise gleichmäßig zueinander bzw. symmetrisch zur Linie B, gemäß Figur 3, angeordnet werden.

Die Einlage 7, entsprechend Figur 3, besteht aus einem Stoff, insbesondere einem Vliesstoff 11, wobei als Saugkern 12 ein Zellstoffkissen 14 vorgesehen ist. Der Saugkern 12 ist zentriert in der Einlage 7 angeordnet und wird von dem Vliesstoff 11 umrandet, wobei an den Beinabschlüssen 8 erfindungsgemäß Vliesstoffflügel 20 vorgesehen sind, die einen zusätzlichen Auslaufschutz bewirken. Die Vliesstoffflügel 20 sind bevorzugt gerafft, so dass ein erhöhter Auslaufschutz gewährleistet wird.
In dem Ausführungsbeispiel gemäß Figur 3 befinden sich die Vliesstoffflügel 20 auf der Höhe der Saugkissenleisten 13.

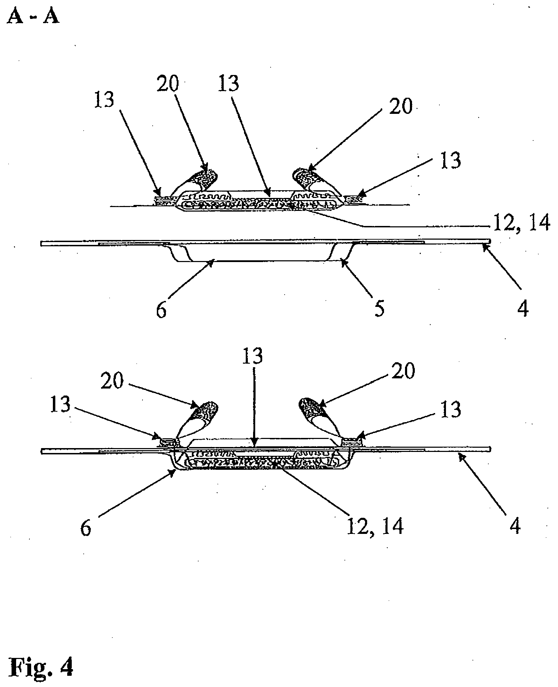

In Figur 4a ist die Einlage 7 im Schnitt A-A, entsprechend Figur 3, dargestellt. Des Weiteren ist in Figur 4a das Außenteil 4 des Hygieneartikels 1 mit integriertem Innenteil 5, welches eine Ausbuchtung 6 zur Aufnahme der saugfähigen Einlage 7 aufweist, gezeigt. In dem Ausführungsbeispiel gemäß Figur 4a sind die Vliesstoffflügel 20, welche an der Einlage 7 angebracht sind und einen zusätzlichen Dichtigkeitsabschluss bewirken, ersichtlich. Zudem sind in Figur 4a die Saugkissenleisten 13 erkennbar, die die dreidimensional-wannenartige Form der Einlage 7 schaffen.

Die Synergie der Einlage 7 und Überhose 4 ist in Figur 4b dargestellt. Die Einlage 7 wird in die Ausbuchtung 6 des Innenteils 5 eingebracht, wobei aus Figur 4b erkennbar ist, dass die Gestaltung der Ausbuchtung 6 des Innenteils 5 an die Form der Einlage 7 angepasst ist. Zudem sind in dem Ausführungsbeispiel gemäß Figur 4b die Saugkissenleisten 13, die das Durchdringen der Körperflüssigkeiten in den Außenteil 4 des Hygieneartikels 1 verhindern und ein Auffangen der festen Fäkalien ermöglichen, dargestellt.
Des Weiteren sind in Figur 4b die Saugkissenleisten 13 in aufgeklapptem Zustand dargestellt. Die Saugkissenleisten 13 werden beim Einbringen der Einlage 7 in die Ausbuchtung 6 des Innenteils 5 des Hygieneartikels 1 aufgeklappt, so dass eine dreidimensional-wannenartige Struktur entsteht. Die Vliesstoffflügel 20, welche sich an der Einlage 7 befinden, führen zu einer zusätzlichen Wandung, die ein Durchdringen der Körperflüssigkeiten in den Außenteil 4, insbesondere im kritischen Beinabschlussbereich 8, verhindern.

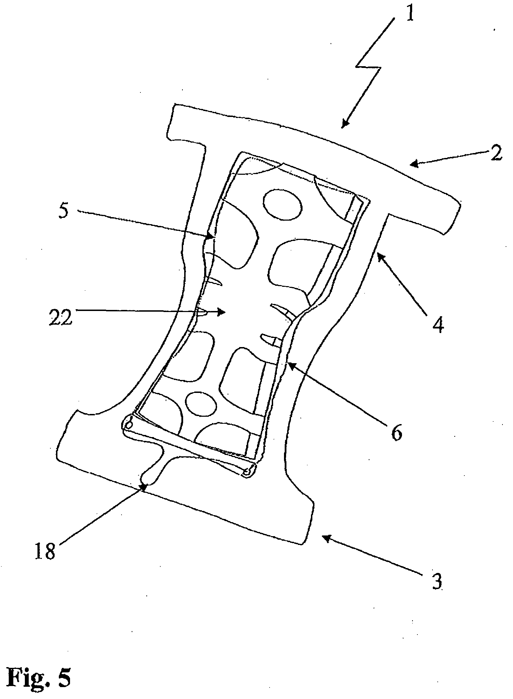

In Figur 5 wird der Hygieneartikel 1 in vereinfachter Darstellung gezeigt. Im Ausführungsbeispiel gemäß Figur 5 ist die Einstellvorrichtung 18, am Rand der Ausbuchtung 6 im integrierten Innenteil 5, zu sehen. Hierbei handelt es sich erfindungsgemäß um einen regulierbaren Gummizug 18, insbesondere einen Gummi-Kordelzug. Durch diese Einstellvorrichtung 18 kann die Ausbuchtung 6 eine an die Form der Einlage 7 angepasste Ausgestaltung einnehmen und vom Träger individuell reguliert werden.

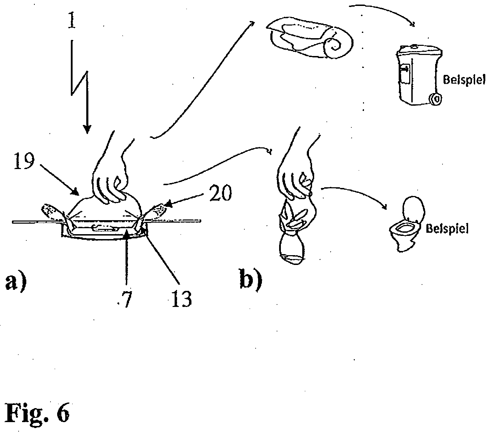

Eine Schnittansicht des Hygieneartikels 1 mit Außenteil 4 und integriertem Innenteil 5, wobei das Innenteil 5 eine Ausbuchtung 6 aufweist, in welche eine saugfähige Einlage 7 zur Aufnahme von Körperflüssigkeiten bzw. festen Fäkalien eingebracht ist, wird in Figur 6 dargestellt. Zudem ist in Figur 6 der Mechanismus zur Entfernung von festen Fäkalienrückständen vereinfacht illustriert. Die oberste Schicht der Einlage 7 weist vorteilhafterweise Fäden 19 auf, die den Stoff 11 der Einlage, insbesondere den Vliesstoff 11, so zusammenziehen, dass der Nutzer nicht in Kontakt mit den Körperflüssigkeiten kommt. Die oberste Schicht der Einlage 7, die aus biologisch abbaubaren Materialien besteht, wobei diese eine begrenzte Nässefestigkeit für die Dauer der Nutzung aufweisen, kann so ohne Probleme in der Toilette entsorgt werden. Das Außenteil 4 des Hygieneartikels 1, mit integriertem Innenteil 5, kann über den Hausmüll entsorgt werden.

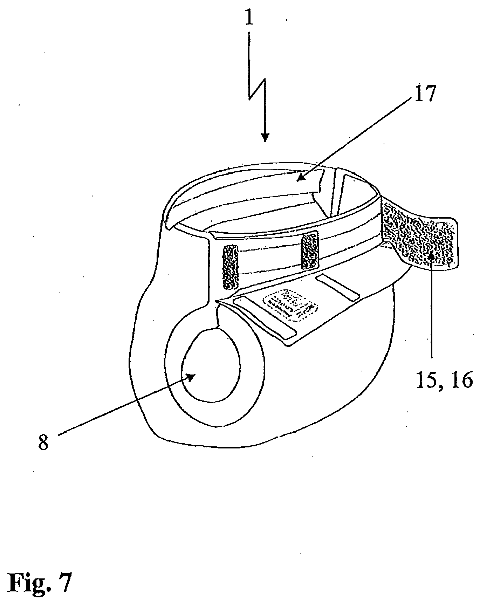

Ein Hygieneartikel 1, entsprechend der Erfindung, ist auch in Figur 7 dargestellt. In diesem Ausführungsbeispiel ist die Überhose 4 zu sehen, wie sie am Träger befestigt wird. Dazu weist die Überhose 4 einen Verstell- und Feststellmechanismus 15 auf, so dass der Hygieneartikel 1 optimal an den Träger angepasst werden kann. Der Verschluss befindet sich vorderseitig an der Überhose 4.

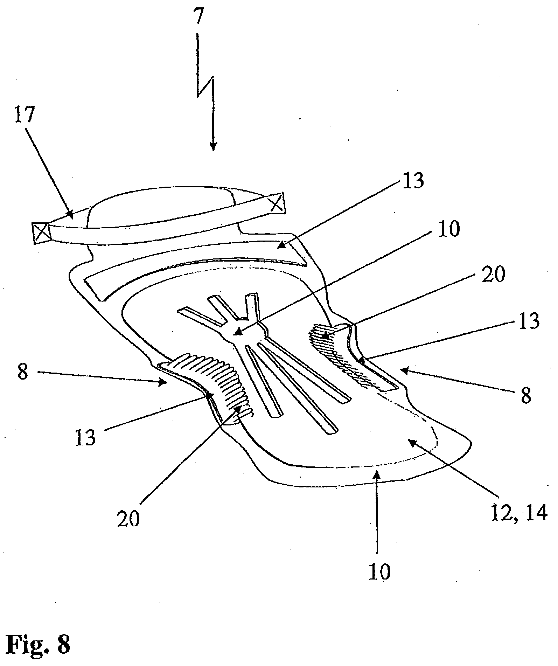

Im Ausführungsbeispiel gemäß Figur 8 ist schematisch die Position der Verbindungselemente 17 an der Einlage 7 dargestellt. Die Verbindungselemente 17 werden mindestens am hinteren Abschnitt 3, innenseitig, der Überhose 4 angeordnet. Allerdings können im Rahmen der Erfindung auch mehrere solcher Verbindungselemente 17 angeordnet sein, wobei diese am hinteren Abschnitt 3, am vorderen Abschnitt 2 oder am hinteren 3 und vorderen Abschnitt 2, jeweils innenseitig, angeordnet sein können.

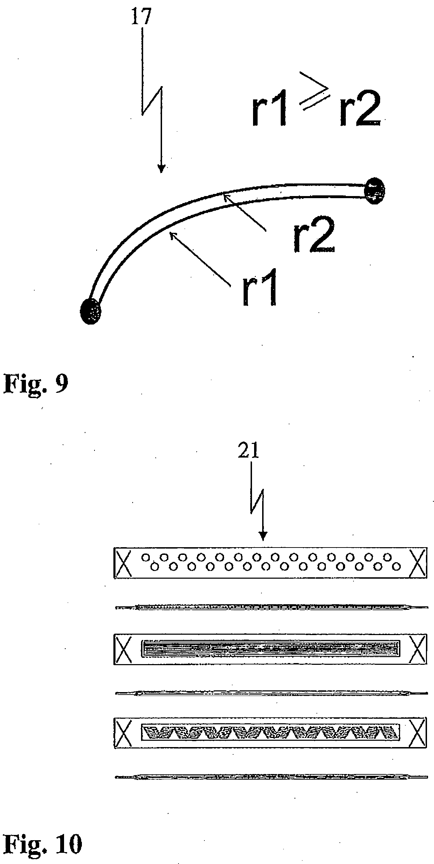

Die Verbindungselemente 17 sind im Außenteil 4 des Hygieneartikels 4 integriert und werden im Ausführungsbeispiel gemäß Figur 9 aus zwei Kurven mit unterschiedlichem Durchmesser r1 und r2 gebildet, wobei r1 ≥ r2 gilt.

Um einen optimalen Halt der Einlage 7 im integrierten Innenteil 5 des Außenteils 4 des Hygieneartikels 1 zu ermöglichen, wird erfindungsgemäß vorgeschlagen, die Verbindungselemente 17 zusätzlich mit Stoppern 21 auszustatten. Diese weisen vorzugsweise weiches Gummimaterial auf und sind in verschiedenen Ausführungsformen, gemäß Figur 10, möglich. Selbstverständlich sind im Rahmen der Erfindung auch weitere Ausführungen der Stopper 21 denkbar.

### Bezugszeichenliste

- 1: Hygieneartikel
- 2: vorderer Teil
- 3: hinterer Teil
- 4: Außenteil / Überhose
- 5: Innenteil
- 6: Ausbuchtung
- 7: Einlage
- 8: Beinabschnitt
- 9: Biegemitte
- 10: Prägung
- 11: Vliesstoff
- 12: Saugkern
- 13: Saugkissenleisten
- 14: Zellstoffkissen
- 15: Einstell-/ Verstellmechanismus
- 16: Verschluss
- 17: Verbindungselemente
- 18: Einstellvorrichtung der Ausbuchtung
- 19: Fäden der Einlage
- 20: Vliesstoffflügel
- 21: Stopper
- 22: Rippung
- B: Linie

## Patentansprüche

1. Hygieneartikel aus biologisch abbaubaren Materialien, bestehend aus einem Innenteil (5) und einem flüssigkeitsabweisenden Außenteil (4), wobei der Innenteil (5) eine Ausbuchtung (6) zur Aufnahme einer saugfähigen Einlage (7), insbesondere eines Saugkissens (12), aufweist, weiterhin die Einlage (7) lösbar im Innenteil (5) angeordnet und das Innenteil (5) integraler Bestandteil des Außenteiles (4) ist, wobei
die Ausbuchtung (6) eine an die Form der Einlage (7) angepasste Gestaltung besitzt und die von der Ausbuchtung (6) aufgenommene Einlage (7) dreidimensional-wannenartig ausgeführt ist,
**dadurch gekennzeichnet, dass** die dreidimensional-wannenartige Form der Einlage (7) durch Saugkissenleisten (13) erzeugt wird.

2. Hygieneartikel nach Anspruch 1,
**dadurch gekennzeichnet, dass**
die Ausbuchtung (6), mindestens im Randbereich, eine elastische Rippung aufweist.

3. Hygieneartikel nach Anspruch 1 oder 2,
**dadurch gekennzeichnet, dass**
die Ausbuchtung (6) am Rand eine Einstellvorrichtung (18), insbesondere einen regulierbaren Gummizug, aufweist.

4. Hygieneartikel nach einem der vorhergehenden Absprüche,
**dadurch gekennzeichnet, dass**
das Außenteil (4) eine Hose ist.

5. Hygieneartikel nach Anspruch 4,
**dadurch gekennzeichnet, dass**
die Hose (4) aus atmungsaktivem Membranstoffverbund besteht.

6. Hygieneartikel nach Anspruch 4 oder 5,
**dadurch gekennzeichnet, dass**
die Hose (4) aus einem oder aus mehreren Teilen besteht.

7. Hygieneartikel nach einem der vorhergehenden Ansprüche,
**dadurch gekennzeichnet, dass**
die Saugkissenleisten (13) klappbar sind.

8. Hygieneartikel nach einem der vorhergehenden Ansprüche,
**dadurch gekennzeichnet, dass**
die Einlage (7) seitliche Ausformungen (20), insbesondere Vliesstoffflügel (20), aufweist.

9. Hygieneartikel nach Anspruch 8,
**dadurch gekennzeichnet, dass**
die Vliesstoffflügel (20) gerafft sind.

10. Hygieneartikel nach einem der vorhergehenden Ansprüche,
**dadurch gekennzeichnet, dass**
die Einlage (7) einen Saugkern (12), bestehend aus mindestens einem Zellstoffkissen (14), aufweist.

11. Hygieneartikel nach Anspruch 10,
**dadurch gekennzeichnet, dass**
in dem Zellstoffkissen (14) eine Prägung (10) eingebracht ist.

12. Hygieneartikel nach Anspruch 10 oder 11,
**dadurch gekennzeichnet, dass**
der Saugkern (12) eine vordefinierte Biegung aufweist.

13. Hygieneartikel nach einem der vorhergehenden Ansprüche,
**dadurch gekennzeichnet, dass**
die Einlage (7) Fäden (19) aufweist, die so angeordnet sind, dass mindestens
die oberste Schicht der Einlage (7) mittels der Fäden (19) zusammenfaltbar ist.

14. Hygieneartikel nach einem der vorhergehenden Ansprüche,
**dadurch gekennzeichnet, dass**
das Außenteil (4) Verbindungselemente (17) aufweist, die die Einlage (7) fixieren.

## Claims

1. Sanitary article made of biodegradable materials, composed of an inner part (5) and a liquid-repellent outer part (4), wherein the inner part (5) comprises a convexity (6) for accommodating an absorbent insert (7), optionally an absorbent pad (12), the insert (7) furthermore being arranged in the inner part (5) to be detachable, and the inner part (5) being an integral component of the outer part (4), wherein the convexity (6) has a design adapted to the shape of the insert (7), and the insert (7) accommodated by the convexity (6) is of a three-dimensional trough-like realization,
**characterized in that**
the three-dimensional trough-like shape of the insert (7) is generated by absorbent pad's strips (13).

2. Sanitary article according to claim 1,
**characterized in that**
the convexity (6) has an elastic ribbing at least in the edge area.

3. Sanitary article according to claim 1 or 2,
**characterized in that**
the convexity comprises on the rim an adjusting device (18), optionally an adjustable elastic band.

4. Sanitary article according to any one of the preceding claims,
**characterized in that**
the outer part (4) is a pair of pants.

5. Sanitary article according to claim 4,
**characterized in that**
the pants (4) are comprised of a breathable membrane fabric composite.

6. Sanitary article according to claim 4 or 5,
**characterized in that**
the pants (4) are comprised of one or more parts.

7. Sanitary article according to any one of the preceding claims,
**characterized in that**
the absorbent pad's strips (13) are foldable.

8. Sanitary article according to any one of the preceding claims,
**characterized in that**
the insert (7) has lateral protrusions (20) such as wings of nonwoven material.

9. Sanitary article according to claim 8,
**characterized in that**
the wings of nonwoven material are gathered.

10. Sanitary article according to any one of the preceding claims,
**characterized in that**
the insert (7) comprises an absorbent core (12) comprised of at least one cellulose pad (14).

11. Sanitary article according to claim 10,
**characterized in that**
an imprint (10) is introduced into the cellulose pad (14)

12. Sanitary article according to claim 10 or 11,
**characterized in that**
the absorbent core (12) has a predefined bending.

13. Sanitary article according to any one of the preceding claims,
**characterized in that**
the insert (7) comprises filaments (19) which are arranged such that at least the uppermost layer of the insert (7) is foldable by means of the filaments (19).

14. Sanitary article according to any one of the preceding claims,
**characterized in that**
the outer part (4) comprises connecting elements (17) fixing the insert (7).

## Revendications

1. Article d'hygiène en matériau biologiquement dégradable, constitué d'une partie intérieure (5) et d'une partie extérieure (4) hydrofuge, dans lequel la partie intérieure (5) comporte un creux (6) pour la réception d'un insert absorbant (7), en particulier d'un coussin absorbant (12), dans lequel l'insert (7) est en outre agencé de façon détachable dans la partie intérieure (5) et la partie intérieure (5) est une composante intégrale de la partie extérieure (4), dans lequel le creux (6) possède une conformation adaptée à la forme de l'insert (7) et l'insert (7) reçu par le creux (6) est réalisé de manière tridimensionnelle et semblable à une cuvette,
**caractérisé en ce que** la forme tridimensionnelle semblable à une cuvette de l'insert (7) est engendrée par des barrettes (13) du coussin absorbant.

2. Article d'hygiène selon la revendication 1,
**caractérisé en ce que** le creux (6) comporte un nervurage élastique, au moins dans la zone de bordure.

3. Article d'hygiène selon la revendication 1 ou 2,
**caractérisé en ce que** le creux (6) comporte en bordure un dispositif de réglage (18), en particulier un lacet en caoutchouc susceptible d'être régulé.

4. Article d'hygiène selon l'une des revendications précédentes,
**caractérisé en ce que** la partie extérieure (4) est une culotte.

5. Article d'hygiène selon la revendication 4,
**caractérisé en ce que** la culotte (4) est constitué d'un composite de membranes respirant.

6. Article d'hygiène selon la revendication 4 ou 5,
**caractérisé en ce que** la culotte (4) est composée d'une seule pièce ou de plusieurs pièces.

7. Article d'hygiène selon l'une des revendications précédentes,
**caractérisé en ce que** les barrettes (13) du coussin absorbant sont rabattables.

8. Article d'hygiène selon l'une des revendications précédentes,
**caractérisé en ce que** l'insert (7) comporte des saillies latérales (20), en particulier des volets (20) en non-tissé.

9. Article d'hygiène selon la revendication 8,
**caractérisé en ce que** les volets (20) en non-tissé sont froncés.

10. Article d'hygiène selon l'une des revendications précédentes,
**caractérisé en ce que** l'insert (7) comprend un noyau absorbant (12), constitué d'au moins un coussin en cellulose (14).

11. Article d'hygiène selon la revendication 10,
**caractérisé en ce qu'**un matriçage (10) est pratiqué dans le coussin en cellulose (14).

12. Article d'hygiène selon la revendication 10 ou 11,
**caractérisé en ce que** le noyau absorbant (12) comporte une courbure prédéfinie.

13. Article d'hygiène selon l'une des revendications précédentes,
**caractérisé en ce que** l'insert (7) comprend des fils (17) qui sont agencés de telle façon que la couche la plus supérieure de l'insert (7) au moins est susceptible d'être plissée au moyen des fils (19).

14. Article d'hygiène selon l'une des revendications précédentes,
**caractérisé en ce que** la partie extérieure (4) comprend des éléments de liaison (17) qui fixent l'insert (7).
